# EUROPÄISCHE PATENTSCHRIFT

(11) **EP 0 781 134 B1**
(45) Veröffentlichungstag und Bekanntmachung des Hinweises auf die Patenterteilung: **24.03.2004**
(21) Anmeldenummer: 95932020.1
(22) Anmeldetag: 09.09.1995
(51) Int. Cl.: A61K 31/46, A61K 9/70

(54) **SCOPOLAMINPFLASTER**
SCOPOLAMINE PLASTER
EMPLATRE A LA SCOPOLAMINE

(30) Priorität: 16.09.1994 DE 4433004; 31.10.1994 DE 4438989
(43) Veröffentlichungstag der Anmeldung: 02.07.1997
(73) Patentinhaber: LTS Lohmann Therapie-Systeme AG, 56626 Andernach (DE)
(72) Erfinder: MÜLLER, Walter, D-56564 Neuwied (DE)
(74) Vertreter: Flaccus, Rolf-Dieter, Dr.
(86) Internationale Anmeldenummer: PCT/EP1995/003555
(87) Internationale Veröffentlichungsnummer: WO 1996/008251

(56) Entgegenhaltungen:
- EP-A- 0 052 074
- EP-A- 0 304 227
- EP-A- 0 356 382
- EP-A- 0 387 693
- EP-A- 0 582 727

## Beschreibung

Scopolamin ist eine bekannte Substanz, die in einem vermarkteten Pflastersystem transdermal mit systemischer Wirkung verabreicht wird.Scopolamin ist ein sogenanntes Antiemetikum und wird bevorzugt eingesetzt zur Vermeidung von Übelkeit und Erbrechen beispielsweise als Folge von bei Reisen auftretenden wiederholten passiven Veränderungen des Gleichgewichts.

Der therapeutische Vorteil der transdermalen Verabreichung ist, daß die Wirkstoffzufuhr langsam und durch das transdermale System gesteuert erfolgt. Es ist dadurch möglich, das recht enge therapeutische Fenster für Scopolamin zuverlässig zu treffen und so ein erseits therapeutisch effektive Plasmaspiegel zu erreichen, ohne andererseits die mit einer Überdosierung verbundenen Nebenwirkungen befürchten zu müssen.
Der Aufbau des vermarkteten Systems ist in der US 3,797,494 beschrieben. Es besteht im wesentlichen aus einer Rückschicht, einem Wirkstoffreservoir, einer mikroporösen Membran, einer ebenfalls wirkstoffhaltigen Hautkleberschicht und einer vor Gebrauch zu entfernenden Schutzfolie. Das Reservoir und die Hautkleberschicht sind aufgebaut aus einer Mischung von Polyisobutylenen mit verschiedenem Molekulargewicht und einem Mineralöl. Der Wirkstoff ist in dieser Mischung als viskose Flüssigkeit dispergiert. Ein transdermales System, dessen wirkstoffhaltigen Teile auf dieser Basis aufgebaut sind, zeigt jedoch gravierende Nachteile. Unter bestimmten Bedingungen kommt es zu spontanen Kristallisationen, die die Bioverfügbarkeit des Wirkstoffs im Pflaster negativ beeinflussen.
In der US 4 832 953 sind die Details dieser Instabilität ausführlich geschildert. Es wird eine Methode beschrieben, wie durch nachträgliche Hitzebehandlung des schon verpackten Pflasters die Kristallisation verhindert werden kann. Entsprechend den Angaben in dieser Patentschrift ist es vorallem das Scopolaminhydrat, das auskristallisiert.

Es ist ein erheblicher Nachteil, wenn unter bestimmten, nur schlecht zu definierenden Bedingungen, der in einer Arzneiform enthaltende Wirkstoff zu einer nicht vorhersagbaren Zeit nach der Herstellung seine Aggregatform unter Beeinflussung der Bioverfügbarkeit wechselt.

In EP-A-0 387 693 wurden transdermale Systeme zur gesteuerten Verabreichung an die Haut beschrieben, wobei das Polymermaterial der Wirkstoffmatrix u. a. aus Polyacrylsäureestern und Copolymerisaten davon aufgebaut sein kann. In einer Beispielformulierung wurde ein Wirkstoffreservoir auf der Basis eines Polyacrylharzklebers hergestellt; als Wirkstoff wurde Scopolamin-Base verwendet. Es werden keine Angaben zum Lösevermögen der Matrixpolymere für den Wirkstoff Scopolamin-Base gemacht.

Ausgehend von diesem Stand der Technik liegt der Erfindung die Aufgabe zugrunde, eine alternative Polymerformulierung für den Wirkstoff Scopolamin-Base anzugeben, die frei ist von den in oben zitierten Patentschriften beschriebenen gravierenden Nachteilen .

Die Lösung der Aufgabe gelingt bei einem transdermalen therapeutischen System der im Oberbegriff von Anspruch 1 genannten Art mit der Erfindung dadurch, daß die wirkstoffhaltigen Schichten des Pflasters als Basispolymer Copolymere von Acrylsäure- bzw. Methacrylsäurederivaten und Scopolamin-Base in einer Konzentration enthalten, die 70-100 % der Sättigungslöslichkeit, bevorzugt 85-100 % der Sättigungslöslichkeit im Wirkstoffreservoir entspricht.

Weitere Ausgestaltungen der Erfindung sind entsprechend den Unteransprüchen vorgesehen.

Scopolamin-Base ist gemäß Literaturangaben eine viskose Flüssigkeit. Es ist jedoch für den Fachmann schwer zu verstehen, daß eine relativ polare Substanz mit einem Molekulargewicht von 303,35 in ihrem stabilen Aggregatzustand eine Flüssigkeit sein soll. Dennoch gelingt es unter entsprechenden Bedingungen, Scopolamin-Base zu kristallisieren. Man erhält einen weißen Festkörper mit einem Schmelzpunkt von ca. 68°C. Daraus ergibt sich zwingend, daß in Pflastern gemäß dem Stand der Technik der Wirkstoff in einer instabilen Modifikation enthalten ist. So ist es sehr wahrscheinlich, daß nicht das Hydrat der Scopolamin-Base die auskristallisierende Spezies ist, sondern daß es sich um eine Phasenumwandlung von flüssig nach fest der Scopolamin-Base selbst handelt.
Da flüssige Scopolamin-Base im Vergleich zur kristallinen Scopolamin-Base eine Modifikation mit erhöhtem Energiegehalt repräsentiert, ergibt sich aus den dem Fachmann bekannten Gesetzesmäßigkeiten, daß Scopolamin-Base im Polymer selbst in einer die Sättigungskonzentration Übersteigenden Konzentration gelöst ist. Die Kristallisation kann damit nicht nur im dispergierten Teil des Wirkstoffs, sondern auch im Polymer selbst stattfinden.

Die Erfindung gibt mit Vorteil eine neue Formulierung der das Scopolamin enthaltenden Basispolymere an, durch welche die bekannten Nachteile für die Leistungsfähigkeit des Pflasters vermieden werden. Dadurch, daß erfindungsgemäß Polymere verwendet werden, die den Wirkstoff komplett gelöst in Konzentrationen unterhalb oder höchstens gleich der Sättigungskonzentration enthalten, wird eine Instabilität des Wirkstoffs Scopolamin und dessen Kristallisation im Pflaster mit großem Vorteil vermieden. Solche Polymere müssen hierfür ein gegenüber den Formulierungen entsprechend dem Stand der Technik erhöhtes Lösevermögen für Scopolamin-Base besitzen.
Als geeignete Polymere erwiesen sich selbstklebende Copolymere auf der Basis von Polyacrylsäuren und ihren Derivaten. In solchen Polymeren hat Scopolamin-Base eine von der genauen Zusammensetzung der Copolymere und der Art und Menge der zugesetzten Hilfsstoffe abhängige Löslichkeit zwischen etwa 10 und 20 % (g/g). Dies ist mehr als ausreichend, um in einem Pflaster mit einer Größe von ca. 2,5 cm² und einer bei transdermalen Systemen üblichen Dicke, die beispielsweise für eine Dreitagesbehandlung zur Prophylaxe von Reisebeschwerden notwendige Menge Wirkstoff unterzubringen.
Die Eigenschaften von solchen Polyacrylatklebern können durch die Wahl der zu ihrer Herstellung verwendeten Monomere und des durch die Polymerisationsbedingungen vorherbestimmbaren Molekulargewichts bezüglich der Klebkraft, der Kohäsion und der Löseeigenschaften variiert werden. Wichtig im Sinne dieser Erfindung ist vor allem das Lösevermögen für Scopolamin-Base. Da Scopolamin-Base eine relativ polare Substanz ist, kommen deshalb vor allem solche Polyacrylatkleber in Frage, die über polare funktionelle Gruppen verfügen. Als solche in Polyacrylatklebern übliche Gruppen seien beispielsweise Carboxylgruppen, Hydroxylgruppen und Aminogruppen genannt. Entsprechende Monomere, die zur Einführung dieser Gruppen in den Kleber dienen können, sind zum Beispiel Methacrylsäure, Acrylsäure, Halbester von Diolen mit Acyrl- und Methacyrlsäure und Ester beider Säuren mit Aminoalkoholen.

Polyacrylatkleber sind kompatibel mit einer Vielzahl von niedermolekularen Substanzen. Der Zusatz von solchen Substanzen kann benutzt werden, um das Lösevermögen von gegebenen Polyacrylatklebern im Sinne dieser Verbindung zu modifizieren. Im praktischen Sinne bedeutet dies, daß Polyacrylatkleber, die über eine ungenügende Anzahl von polaren Gruppen verfügen, mit einem Zusatz von relativ polaren Hilfsstoffen, und Polyacrylatkleber mit einer zu hohen Anzahl von polaren Gruppen mit relativ unpolaren Hilfsstoffen versehen werden müssen. Da die thermodynamische Aktivität von Wirkstoffen nicht von der absoluten Konzentration, sondern vielmehr von dem Verhältnis der aktuellen Konzentration zur Sättigungslöslichkeit abhängt, ist die letztere Möglichkeit wichtig, um bei einem Polyacrylatkleber mit einer zu hohen Löslichkeit für den Wirkstoff durch eine Verminderung der Löslichkeit für Scopolamin-Base Wirkstoff einzusparen. Unter diesen Gesichtspunkten ist im Sinne dieser Erfindung eine Sättigungslöslichkeit von Scopolamin-Base in dem Kleber bzw. in der Mischung Kleber/Hilfsstoff von 10 - 30 Gewichtsprozenten als optimal anzusehen.

Da Scopolamin-Base eine realtiv polare Substanz ist, kann vor allem der Zusatz von flüssigen Kohlenwasserstoffen benutzt werden, um die Sättigungslöslichkeit zu erniedrigen. Als besonders geeignet hat sich dafür Dioctylcyclohexan erwiesen.
Als polarere Substanzen zur Erhöhung der Sättigungslöslichkeit können vor allem Fettsäurem, Fettalkohole, Polyethylen- bzw. Polypropylenglykol, Derivate des Glycerins und Dexpanthenol eingesetzt werden. Als besonders geeignet haben sich Fettsäuren wie die Ölsäure erwiesen.

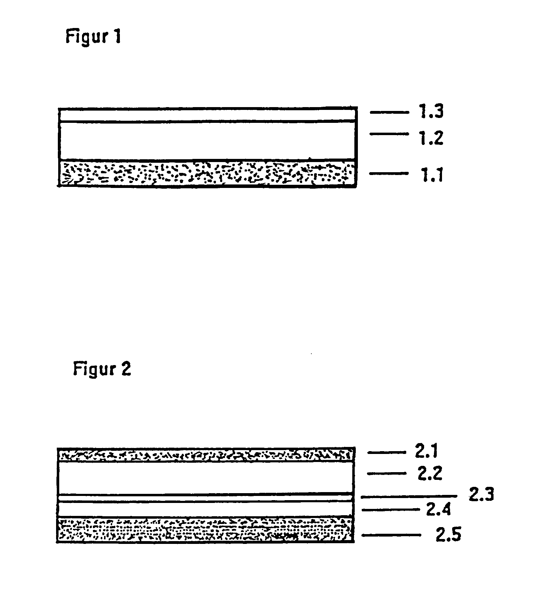

Auf Basis dieser Kleber bzw. Kleber/Hilfsstofformulierungen können sowohl sogenannte transdermale Matrixsysteme als auch Membransysteme hergestellt werden, die sich in aussagekräftigen in-vitro-Permeationsversuchen an Menschenhaut als bioäquivalent mit vermarkteten Konkurrenzprodukten erwiesen haben. Der Aufbau solcher transdermaler Systeme ist in Figur 1 und 2 dargestellt.
Ein Matrixsystem ist konstruktiv das einfachste transdermale System. Es besteht aus einer im wesentlichen für den Wirkstoff und die Hilfsstoffe undurchlässigen Rückschicht (1.3), einer wirkstoffhaltigen, selbstklebenden Polymerformulierung (1.2) und einer vor Gebrauch zu entfernenden Schutzfolie (1.1). Die für die Rückschicht und die Schutzschicht in Frage kommenden Materialien sind dem Fachmann bekannt. Praktisch universell sind Folien auf der Basis von Polyethylent erephtalat, wobei die Schutzfolie zumindest auf ihrer Kontaktseite zum Kleber zusätzlich silikonisiert ist, um das Ablösen des Pflasters zu erleichtern. Bei zu geringer Haftklebrigkeit der Polymerformulierung kann diese zur Haut hin mit einer zusätzlichen Haftkleberschicht ausgestaltet sein, die hier nicht dargestellt ist.

Ein Membransystem besteht aus einer Rückschicht (2.5), einem Wirkstoffreservoir (2.4), der Membran (2.3), einer Hautkontaktschicht (2.2) und einer vor Verwendung zu entfernenden Schutzschicht (2.1).
Die Wirkstoffreservoirschicht und die Hautkontaktschicht können die gleiche oder eine unterschiedliche Zusammensetzung aufweisen. Wichtig im Sinne dieser Erfindung ist lediglich, daß zumindest das Reservoir auf einem Polyacrylatkleber beruht und über ein Lösevermögen für Scopolamin-Base zwischen 10 und 30 Gewichtsprozenten verfügt.
Auch die für eine Membran in Frage kommenden Materialien sind dem Fachmann bekannt. Besonders bewährt haben sich in Kombination mit einem Polyacrylatkleber im Sinne dieser Erfindung Membranen auf der Basis von Copolymeren aus Ethylen und Vinylacetat. Durch den Gehalt an Vinylacetat und die Dicke der Membran läßt sich der Wirkstofffluß durch diese Membran steuern. Je höher der Vinylacetatgehalt und je geringer die Dicke, desto größer ist die Durchlässigkeit der Membran für Scopolamin-Base. Bewährt haben sich Membranen mit einem Vinylacetatgehalt von mindestens 4 % und einer Dicke zwischen 50 und 100 µm. Für ein Pflaster mit einer 5 cm² nicht Übersteigenden Größe haben sich Membranen mit einer Dicke von 50 µm und einem Vinylacetatgehalt von 9 - 20 % besonders bewährt.

In Tabelle 1 und Diagramm 1 sind die Ergebnisse von Permeationsexperimenten unter Verwendung von Matrixsystemen im Sinne dieser Erfindung gezeigt. Die Versuche wurden durchgeführt unter der Benutzung von Franz-Permeationszellen und Verwendung von menschlicher Haut (weibliche Brusthaut aus Brustreduktionsoperation).

In Tabelle 2 und Diagramm 2 sind die Ergebnisse mit Membransystemen im Sinne der Erfindung gezeigt.

**Tabelle 1**

| Permeationsmessungen mit Matrixsystemen gemäß Beispiel 1 und 2 | | | | |
|---|---|---|---|---|
| Formulierung | Kumulierte Menge an durch die Haut permeierte Scopolamin Base in µg/cm² (Mittelwert aus n = 3) nach | | | |
| | 16h | 24h | 40h | 48h |
| Vergleich | 115,8 | 185,1 | 297,4 | 350,7 |
| Beispiel 1 | 118,1 | 208,0 | 349,1 | 403,0 |
| Beispiel 2 | 120,3 | 203,4 | 320,2 | 410,3 |

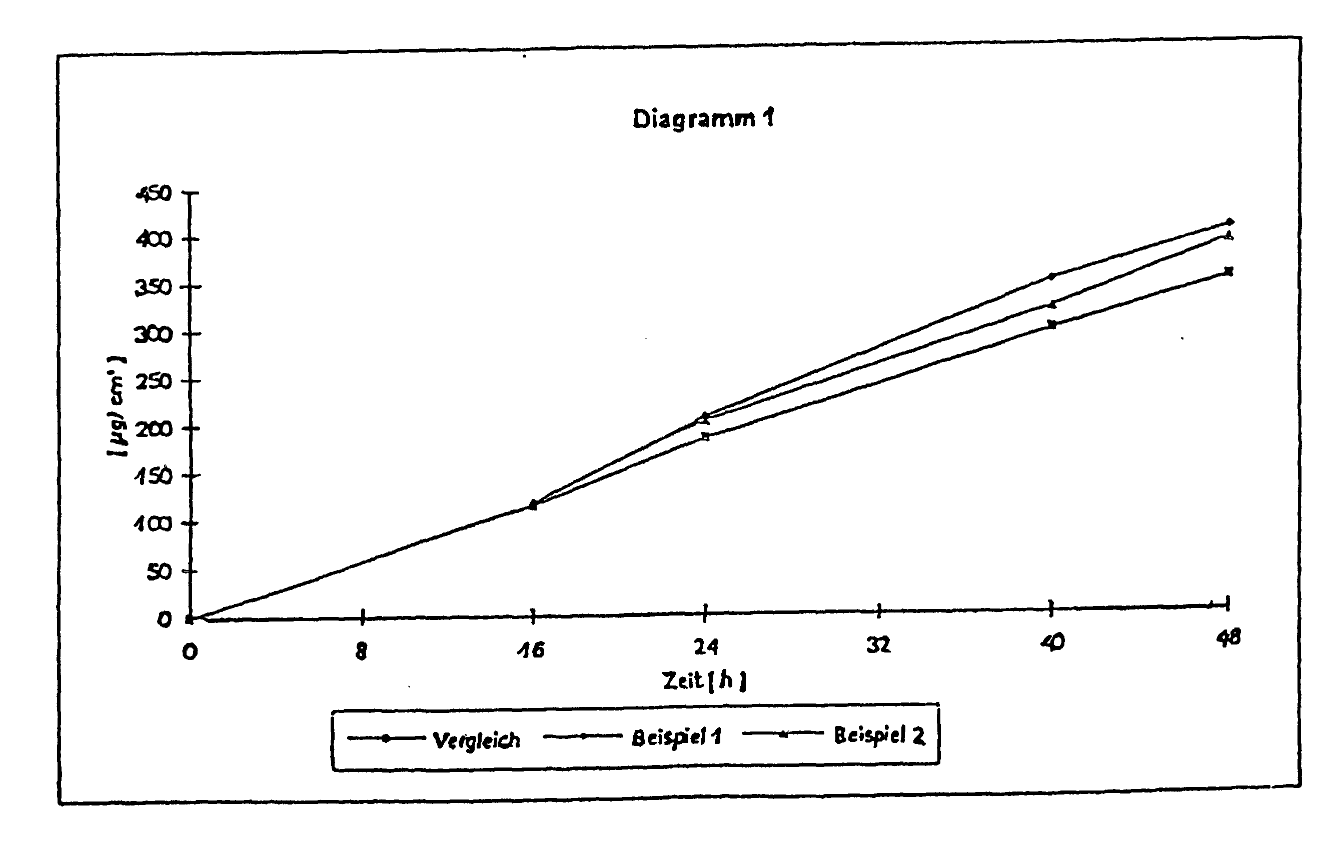

**Tabelle 2**

| Permeationsmessungen mit Membransystemen gemäß Beispiel 3 und 4 | | | | |
|---|---|---|---|---|
| Formulierung | Kumulierte Menge an durch die Haut permeierte Scopolamin Base in ug/cm² (Mittelwert aus n = 3) nach | | | |
| | 16 h | 24 h | 40 h | 48 h |
| Vergleich | 115,8 | 185,1 | 297,4 | 350,7 |
| Beispiel 3 | 129,9 | 209,6 | 311,7 | 350,0 |
| Beispiel 4 | 135,4 | 215,3 | 330,8 | 360,4 |

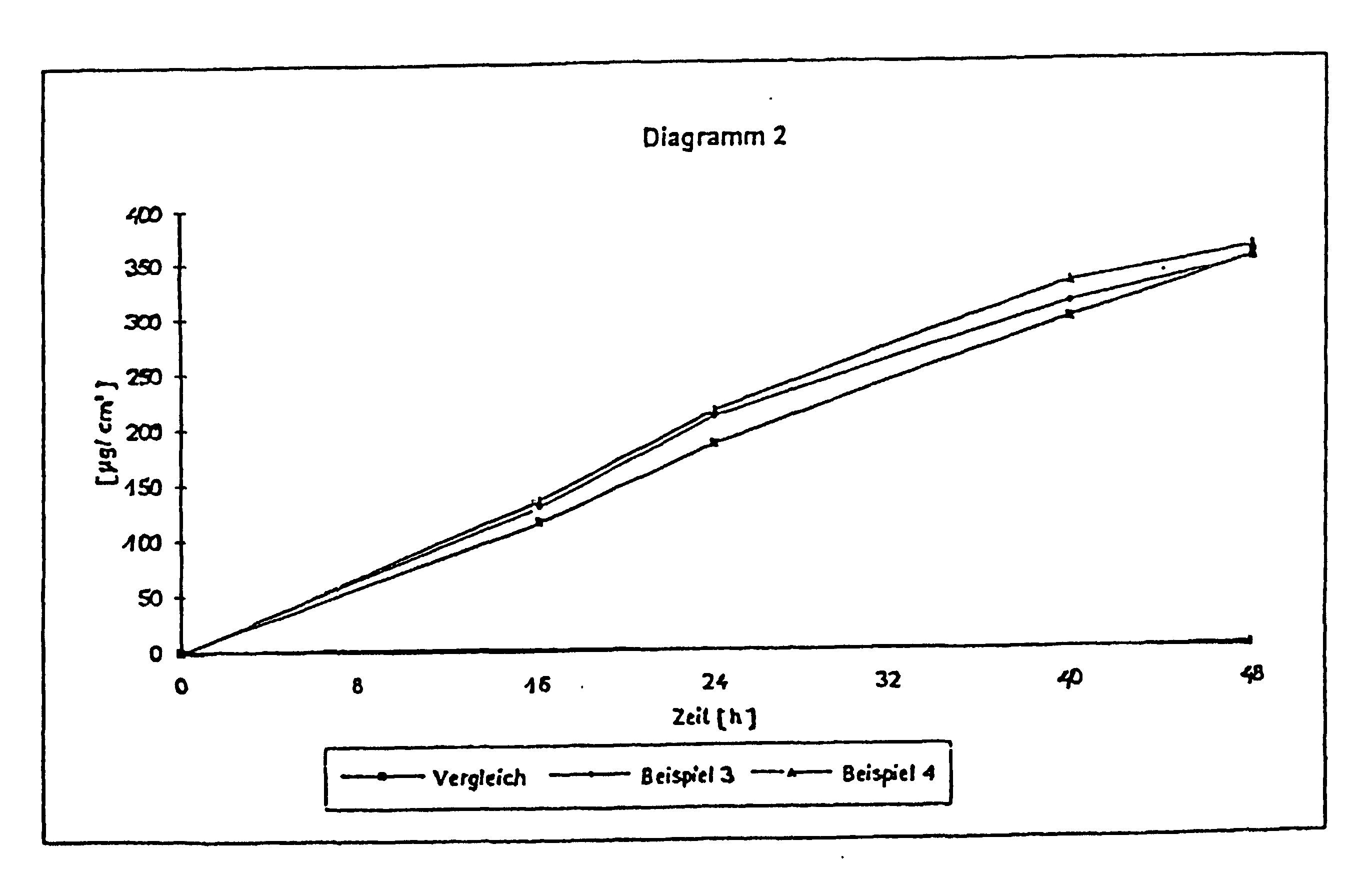

Die Ergebnisse dieser Permeationsexperimente zeigen eindeutig, daß Pflaster im Sinne dieser Erfindung die gleiche Leistungsfähigkeit besitzen wie die Vergleichsmuster, ohne jedoch über deren Nachteile zu verfügen. Da die Wirkstoffkonzentration die Sättigungslöslichkeit nicht übersteigt, besteht keine Gefahr der Rekristallisation. Auch die Bildung von kristallinem Scopolaminhydrat ist praktisch unmöglich, da Polyacrylatformulierungen aufgrund ihrer chemisch-physikalischen Eigenschaften über ein Lösevermögen für Scopolaminhydrat verfügen, das ausreichend ist, um eine Rekristallisation bei den praktisch vorhandenen Wassermengen im Kleber zu verhindern.

### Beispiele

### Beispiel 1

27 g Polyacrylatkleber (Durotak 901-1051, Feststoffgehalt 52 %)
3,4 g Ölsäure
0,12 g Aluminiumacetylacetonat
4,0 g Scopolamin-Base
und 3,7 g Ethanol
werden sorgfältig gemischt und als 200 pm dicker Film auf eine silikonisierte Polyesterfolie beschichtet. Der lösemittelhaltige Film wird 30 Minuten bei 50°C getrocknet und mit einer 23 µm dicken Polyesterfolie abgedeckt. Aus dem Gesamtlaminat werden die einzelnen Pflastersysteme (Fläche 2,5 cm²) ausgestanzt.

### Beispiel 2

27 g Polyacrylatkleber (Durotak 901-1051, Feststoffgehalt 52 %)
3,4 g Oleylalkohol
0,12 g Aluminiumacetylacetonat
3,0 g Scopolamin-Base
und 3,7 g Ethanol
werden sorgfältig gemischt und als 200 µm dicker Film auf eine silikonisierte Polyesterfolie beschichtet. Der lösemittelhaltige Film wird 30 Minuten bei 50°C getrocknet und mit einer 23 µm dicken Polyesterfolie abgedeckt. Aus dem Gesamtlaminat werden die einzelnen Pflastersysteme (Fläche 2,5 cm²) ausgestanzt.

### Beispiel 3

73,6 g Polyacrylatkleber (Durotak 901-1051, Feststoffgehalt 52 %)
9 g Ölsäure
0,38 g Aluminiumacetylacetonat
12,0 g Scopolamin-Base
und 17 g Ethanol
werden sorgfältig gemischt und für die Beschichtungsvorgänge genommen.

### a. Herstellung der Hautkleberschicht

Die Masse wird als 50 µm dicker Film auf eine silikonisierte Polyesterfolie beschichtet. Der lösemittelhaltige Film wird 30 Minuten bei 50°C getrocknet und mit einer 50 µm dicken Membran aus einem Ethylen-Vinylacetat-Copolymer mit einem Vinylacetatgehalt von 9 % abgedeckt.

### b. Herstellung der Reservoirschicht

Die gleiche Masse wird in einem zweiten Beschichtungsvorgang in einer Dicke von 150 µm auf eine weitere silikonisierte Polyesterfolie beschichtet und nach dem Entfernen der Lösemittel mit einer 23 µm dicken Polyesterfolie abgedeckt.

### c. Herstellung des Gesamtlaminats

Die unter b hergestellte Reservoirschicht wird von der Polyesterfolie abgezogen und auf die Membran der unter a hergestellten Hautkleberschicht auflaminiert.

Die Pflastersysteme werden aus dem Gesamtlaminat in einer Größe von 2,5 cm² ausgestanzt.

### Beispiel 4

73,6 g Polyacrylatkleber (Durotak 901-1051, Feststoffgehalt 52 %)
9 g Oleylalkohol
0,38 g Aluminiumacetylacetonat
9,0 g Scopolamin-Base
und 17 g Ethanol
werden sorgfältig gemischt und für die Beschichtungsvorgänge genommen.

Das weitere Vorgehen entspricht Beispiel 3.

## Patentansprüche

1. Transdermales therapeutisches System in Form eines Pflasters mit einem geschichteten Aufbau, bestehend aus einer Rückschicht, einem haftklebenden Wirkstoffreservoir, fallweise einer den Wirkstofffluß steuernden Membran, einer gegebenenfalls zusätzlichen Hautkleberschicht und einer ablösbaren Schutzfolie, enthaltend den Wirkstoff Scopolamin-Base, **dadurch gekennzeichnet, daß** die wirkstoffhaltigen Schichten dieses Pflasters als Basispolymer polare Gruppen aufweisende Copolymere von Acrylsäure- bzw. Methacrylsäurederivaten enthalten, die selbst oder zusammen mit polaren Hilfsstoffen ein Lösungsvermögen für Scopolamin-Base von 10- 30 Gew.-% aufweisen und Scopolamin-Base in einer Konzentration enthalten, die 50-100 % der Sättigungslöslichkeit, bevorzugt 60-100 % der Sättigungslöslichkeit, in der vollständigen Vehikelformulierung entspricht.

2. Transdermales therapeutisches System gemäß Anspruch 1, **dadurch gekennzeichnet, daß** es ein Matrixsystem ist.

3. Transdermales therapeutisches System gemäß Anspruch 1, **dadurch gekennzeichnet, daß** es ein Membransystem ist.

4. Transdermales therapeutisches System nach einem oder mehreren der Ansprüche 1 bis 3, **dadurch gekennzeichnet, daß** die Membran ein Copolymer aus Ethylen und Vinylacetat enthält.

5. Transdermales therapeutisches System nach Anspruch 4, **dadurch gekennzeichnet, daß** die Membran mindestens 4 % vinylacetat enthält.

6. Transdermales therapeutisches System gemäß Anspruch 1 bis 5, dadurch gekennzeichent, daß die wirkstoffhaltigen Schichten des Pflasters Kohlenwasserstoffe, bevorzugt Dioctylcyclohexan, zur Verminderung der Sättigungslöslichkeit für Scopolamin-Base, enthalten.

7. Transdermales therapeutisches System nach einem oder mehreren der Ansprüche 1 bis 6, **dadurch gekennzeichnet, daß** die wirkstoffhaltigen Schichten des Pflasters Fettsäuren oder Fettalkohole zur Erhöhung der Sättigungslöslichkeit, bevorzugt Ölsäure und Oleylalkohol, enthalten.

## Claims

1. A transdermal therapeutic system in the form of a patch having a layered structure, consisting of a backing layer,
a pressure sensitive adhesive active substance reservoir, if needed a membrane controlling the active substance flux, optionally an additional skin adhesive layer, and a removable protective film, and comprising the active substance scopolamine base,
**characterized in that**
the active substance-containing layers of the patch comprise as base polymer polar groups-containing copolymers of acrylic acid or methacrylic acid derivatives, which copolymers, themselves or together with polar auxiliary agents, have a solvency for scopolamine base of 10 - 30%-wt. and comprise scopolamine base in a concentration corresponding to 50 - 100% of the saturation solubility, preferably 60 - 100% of the saturation solubility, in the complete vehicle formulation.

2. The transdermal therapeutic system according to claim 1 **characterized in that** it is a matrix system.

3. The transdermal therapeutic system according to claim 1 **characterized in that** it is a membrane system.

4. The transdermal therapeutic system according to one or several of claims 1 to 3 **characterized in that** the membrane comprises a copolymer of ethylene and vinyl acetate.

5. The transdermal therapeutic system according to claim 4 **characterized in that** the membrane comprises at least 4% of vinyl acetate.

6. The transdermal therapeutic system according to claims 1 to 5 **characterized in that** the active substance-containing layers of the patch comprise hydrocarbons, preferably dioctyl cyclohexane, to decrease the saturation solubility for scopolamine base.

7. The transdermal therapeutic system according to one or several of claims 1 to 6 **characterized in that** the active substance-containing layers of the patch comprise fatty acids or fatty alcohols to increase the saturation solubility, preferably oleic acid and oleyl alcohol.

## Revendications

1. Système thérapeutique transdermique sous forme d'un pansement avec une structure à couches, constitué d'une couche postérieure, d'un réservoir adhésif de substance active, le cas échéant d'une membrane réglant le flux de substance active, le cas échéant d'une couche adhésive supplémentaire sur la peau et d'une feuille de protection détachable, contenant la substance active scopolamine-base, **caractérisé en ce que** les couches contenant la substance active de ce pansement contiennent comme polymère de base des copolymères, présentant des groupes polaires, de dérivés de l'acide acrylique ou méthacrylique, qui présentent eux-mêmes ou avec des adjuvants polaires, un pouvoir de dissolution pour la scopolamine-base de 10 à 30% en poids, et qui contiennent la scopolamine-base en une concentration qui correspond à 50-100% de la solubilité de saturation, de préférence à 60-100% de la solubilité de saturation, dans la formulation totale du véhicule.

2. Système thérapeutique transdermique selon la revendication 1, **caractérisé en ce qu'**il s'agit d'un système à matrice.

3. Système thérapeutique transdermique selon la revendication 1, **caractérisé en ce qu'**il s'agit d'un système à membrane.

4. Système thérapeutique transdermique selon l'une ou plusieurs des revendications 1 à 3, **caractérisé en ce que** la membrane contient un copolymère d'éthylène et d'acétate de vinyle.

5. Système thérapeutique transdermique selon la revendication 4, **caractérisé en ce que** la membrane contient au moins 4% d'acétate de vinyle.

6. Système thérapeutique transdermique selon les revendications 1 à 5, **caractérisé en ce que** les couches contenant la substance active du pansement contiennent des hydrocarbures, de préférence du dioctylcyclohexane, pour diminuer la solubilité de saturation de la scopolamine-base.

7. Système thérapeutique transdermique selon l'une ou plusieurs des revendications 1 à 6, **caractérisé en ce que** les couches contenant la substance active du pansement contiennent des acides gras ou des alcools gras pour augmenter la solubilité de saturation, de préférence de l'acide oléique et de l'alcool oléique.
